# EUROPEAN PATENT APPLICATION

(11) **EP 1 298 220 A1**
(43) Date of publication of application: **02.04.2003**
(21) Application number: 01122689.1
(22) Date of filing: 01.10.2001
(51) Int. Cl.: C12Q 1/68, C12N 15/55, C12N 9/14, C12N 15/12, C07K 14/47, G01N 33/68, A61K 48/00

(54) **Single nucleotide polymorphisms predicting cardiovascular disease**

(71) Applicant: BAYER AG, 51368 Leverkusen (DE)
(72) Inventor: Stropp, Udo, Dr., 42781 Haan (DE); Schwers, Stephan, Dr., 51145 Köln (DE); Reifenberger, Elke, Dr., 53757 St. Augustin-Hangelar (DE); Kallabis, Harald, Dr., 51061 Köln (DE)

(57) **Abstract**

The present invention relates to an isolated polynucleotide encoding a Na⁺/K⁺ ATPase polypeptide useful in methods to identify therapeutic agents useful for treating cardiovascular diseases, the polynucleotide is selected from the group consisting of:

SEQ ID 4 and SEQ ID 5 (baySNP-1765) with allelic variation G in position 240 contained in a functional surrounding like full length cDNA for Na+/K+ ATPase and with or without the Na+/K+ ATPase promotor sequence; and SEQ ID 4 and SEQ ID 5 (baySNP-1765) with allelic variation A in position 240 contained in a functional surrounding like full length cDNA for Na+/K+ ATPase and with or without the Na+/K+ ATPase promotor sequence.

The invention also provides diagnostic methods and kits including antibodies determining whether a human subject is at risk for a cardiovascular disease. The invention provides further polymorphic sequences and other genes.

## Description

### Technical Field

This invention relates to genetic polymorphisms useful for assessing cardiovascular risks in humans, including, but not limited to, atherosclerosis, ischemia/reperfusion, hypertension, restenosis, arterial inflammation, myocardial infarction, and stroke. Specifically, the present invention identifies and describes gene variations which are individually present in humans with cardiovascular disease states, relative to humans with normal, or non-cardiovascular disease states, and/or in response to medications relevant to cardiovascular disease. Further, the present invention provides methods for the identification and therapeutic use of compounds as treatments of cardiovascular disease. Moreover, the present invention provides methods for the diagnostic monitoring of patients undergoing clinical evaluation for the treatment of cardiovascular disease, and for monitoring the efficacy of compounds in clinical trials. Still further, the present invention provides methods to use gene variations to predict personal medication schemes. Additionally, the present invention describes methods for the diagnostic evaluation and prognosis of various cardiovascular diseases, and for the identification of subjects exhibiting a predisposition to such conditions.

### Background of the Invention

Cardiovascular disease is a major health risk throughout the industrialized world.

Cardiovascular diseases include the following disorders of the heart and the vascular system: congestive heart failure, myocardial infarction, ischemic diseases of the heart, all kinds of atrial and ventricular arrhythmias, hypertensive vascular diseases and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in an perfusion which is inadequate to meet the myocardial requirement for oxygen. This group of diseases include stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others).

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis, the most prevalent of vascular diseases, is the principal cause of heart attack, stroke, and gangrene of the extremities, and thereby the principal cause of death. Atherosclerosis is a complex disease involving many cell types and molecular factors (for a detailed review, see Ross, 1993, Nature 362: 801-809 and Lusis, A. J., Nature 407, 233-241 (2000)). The process, in normal circumstances a protective response to insults to the endothelium and smooth muscle cells (SMCs) of the wall of the artery, consists of the formation of fibrofatty and fibrous lesions or plaques, preceded and accompanied by inflammation. The advanced lesions of atherosclerosis may occlude the artery concerned, and result from an excessive inflammatory-fibroproliferative response to numerous different forms of insult. For example, shear stresses are thought to be responsible for the frequent occurrence of atherosclerotic plaques in regions of the circulatory system where turbulent blood flow occurs, such as branch points and irregular structures.

The first observable event in the formation of an atherosclerotic plaque occurs when blood-borne monocytes adhere to the vascular endothelial layer and transmigrate through to the sub-endothelial space. Adjacent endothelial cells at the same time produce oxidized low density lipoprotein (LDL). These oxidized LDLs are then taken up in large amounts by the monocytes through scavenger receptors expressed on their surfaces. In contrast to the regulated pathway by which native LDL (nLDL) is taken up by nLDL specific receptors, the scavenger pathway of uptake is not regulated by the monocytes.

These lipid-filled monocytes are called foam cells, and are the major constituent of the fatty streak. Interactions between foam cells and the endothelial and SMCs which surround them lead to a state of chronic local inflammation which can eventually lead to smooth muscle cell proliferation and migration, and the formation of a fibrous plaque. Such plaques occlude the blood vessel concerned and thus restrict the flow of blood, resulting in ischemia.

Ischemia is a condition characterized by a lack of oxygen supply in tissues of organs due to inadequate perfusion. Such inadequate perfusion can have number of natural causes, including atherosclerotic or restenotic lesions, anemia, or stroke, to name a few. Many medical interventions, such as the interruption of the flow of blood during bypass surgery, for example, also lead to ischemia. In addition to sometimes being caused by diseased cardiovascular tissue, ischemia may sometimes affect cardiovascular tissue, such as in ischemic heart disease. Ischemia may occur in any organ, however, that is suffering a lack of oxygen supply.

The most common cause of ischemia in the heart is atherosclerotic disease of epicardial coronary arteries. By reducing the lumen of these vessels, atherosclerosis causes an absolute decrease in myocardial perfusion in the basal state or limits appropriate increases in perfusion when the demand for flow is augmented. Coronary blood flow can also be limited by arterial thrombi, spasm, and, rarely, coronary emboli, as well as by ostial narrowing due to luetic aortitis. Congenital abnormalities, such as anomalous origin of the left anterior descending coronary artery from the pulmonary artery, may cause myocardial ischemia and infarction in infancy, but this cause is very rare in adults. Myocardial ischemia can also occur if myocardial oxygen demands are abnormally increased, as in severe ventricular hypertrophy due to hypertension or aortic stenosis. The latter can be present with angina that is indistinguishable from that caused by coronary atherosclerosis. A reduction in the oxygen-carrying capacity of the blood, as in extremely severe anemia or in the presence of carboxy-hemoglobin, is a rare cause of myocardial ischemia. Not infrequently, two or more causes of ischemia will coexist, such as an increase in oxygen demand due to left ventricular hypertrophy and a reduction in oxygen supply secondary to coronary atherosclerosis.

The foregoing studies are aimed at defining the role of particular gene variations presumed to be involved in the misleading of normal cellular function leading to cardiovascular disease. However, such approaches cannot identify the full panoply of gene variations that are involved in the disease process, much less identify those which may serve as therapeutic targets for the diagnosis and treatment of various forms of cardiovascular disease.

At present, the only available treatments for cardiovascular disorders are pharmaceutical based medications that are not targeted to an individual's actual defect; examples include angiotensin converting enzyme (ACE) inhibitors and diuretics for hypertension, insulin supplementation for non-insulin dependent diabetes mellitus (NIDDM), cholesterol reduction strategies for dyslipidaemia, anti-coagulants, β blockers for cardiovascular disorders and weight reduction strategies for obesity. If targeted treatment strategies were available it might be possible to predict the response to a particular regime of therapy and could markedly increase the effectiveness of such treatment. Although targeted therapy requires accurate diagnostic tests for disease susceptibility, once these tests are developed the opportunity to utilize targeted therapy will become widespread. Such diagnostic tests could initially serve to identify individuals at most risk of hypertension and could allow them to make changes in lifestyle or diet that would serve as preventative measures. The benefits associated by coupling the diagnostic tests with a system of targeted therapy could include the reduction in dosage of administered drugs and thus the amount of unpleasant side effects suffered by an individual. In more severe cases a diagnostic test may suggest that earlier surgical intervention would be useful in preventing a further deterioration in condition.

It is an object of the invention to provide genetic diagnosis of predisposition or susceptibility for cardiovascular diseases. Another related object is to provide treatment to reduce or prevent or delay the onset of disease in those predisposed or susceptible to this disease. A further object is to provide means for carrying out this diagnosis.

Accordingly, a first aspect of the invention provides a method of diagnosis of disease in an individual, said method comprising determining the genotype of a Na⁺/K⁺ ATPase gene in said individual.

In another aspect, the invention provides a method of identifying an individual predisposed or susceptible to a disease, said method comprising determining the genotype of a Na⁺/K⁺ ATPase gene in said individual.

The invention is of advantage in that it enables diagnosis of a disease or of certain disease states via genetic analysis which can yield useable results before onset of disease symptoms, or before onset of severe symptoms. The invention is further of advantage in that it enables diagnosis of predisposition or susceptibility to a disease or of certain disease states via genetic analysis.

The invention may also be of use in confirming or corroborating the results of other diagnostic methods. The diagnosis of the invention may thus suitably be used either as an isolated technique or in combination with other methods and apparatus for diagnosis, in which latter case the invention provides a further test on which a diagnosis may be assessed.

The present invention stems from using allelic association as a method for genotyping individuals; allowing the investigation of the molecular genetic basis for cardiovascular diseases. In a specific embodiment the invention tests for the polymorphism in an intronic sequence of the Na⁺/K⁺ ATPase gene. The invention demonstrates a link between this polymorphism and predisposition to cardiovascular diseases by showing that allele frequencies extremely differ when individuals with "bad" serum lipids are compared to individuals with "good" serum levels. The meaning of "good and bad" serum lipid levels is defined in Table 1.

Certain disease states would benefit, that is to say the suffering of the patient may be reduced or prevented or delayed, by administration of treatment or therapy in advance of disease appearance; this can be more reliably carried out if advance diagnosis of predisposition or susceptibility to disease can be diagnosed.

### Detailed Description of the Invention

The present invention is based at least in part on the discovery that a specific allele of a polymorphic region of the human sodium-potassium ATPase alpha 2 gene, Na⁺/K⁺ ATPase, is associated with a risk for cardiovascular diseases. The Na⁺/K⁺ ATPase is a heteromeric enzyme comprised of an α and a β subunit. Four α subunits and three β subunits are known with different expression pattern in the human body. As a reference, a list of human Na⁺/K⁺ ATPase subunits and of other genes mentioned in this file is summarized in Table 4 with accession numbers. The Na⁺/K⁺ ATPase is an integral membrane protein that catalyses the transport of three sodium ions out of the cell and two potassium ions into the cell at the expense of one molecule ATP. This enzyme is responsible for the maintenance of a sodium/potassium ion gradient along the cell membrane which is very important for normal cellular physiology. James, P.F. et al. (Molecular Cell, 3, 555-563, 1999) showed that the alpha2 subunit plays also an important role in keeping the calcium ion level low in cardiac muscle cells which is a prerequisite for a healthy condition of the heart and other muscles. Surprisingly, we found a polymorphic site in the alpha2 gene locus that shows a strong correlation between "good and bad" serum lipid levels: 146 patients with "good" serum lipid levels were compared with their respective genotypes of the sodium-potassium ATPase alpha 2 gene to 132 patients with "bad" serum lipid levels.

As SNPs are linked to other SNPs in neighboring genes on a chromosome (Linkage Disequilibrium) those SNPs could also be used as marker SNPs. In a recent publication it was shown that SNPs are linked over 100 kb in some cases more than 150 kb (Reich D.E. et al. Nature 411, 199-204, 2001). The human sodium-potassium ATPase alpha 2 gene is located on Chromosome 1q21-q23 (Oakey R.J. et al. Hum. Molec. Genet. 1, 613-620, 1992). Genes that lie in the above mentioned range near the Na⁺/K⁺ ATPase alpa2 gene (ATP1A2) are potassium inwardly-rectifying channel (KCNJ9 also known as GIRK3), calsequestrin 1 CASQ1), phosphoprotein enriched in astrocytes 15 (PEA15; this protein is also called PED for protein enriched in diabetes and is overexpressed in fibroblasts, skeletal muscle, and adipose tissue from type II diabetics), peroxisomal farnesylated protein (PXF), and coatomer protein complex (COPA). SNPs in these genes could also be linked to the polymorphism of the Na⁺/K⁺ ATPase alpa2 gene and by this being a CVD marker. These associations could be performed as described for the Na⁺/K⁺ ATPase alpa2 gene polymorphism in methods.

### Definitions

For convenience, the meaning of certain terms and phrases employed in the specification, examples, and appended claims are provided below. Moreover, the definitions by itself are intended to explain a further background of the invention.

The term "allele", which is used interchangeably herein with "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.

The term "allelic variant of a polymorphic region of a gene" refers to a region of a gene having one of several nucleotide sequences found in that region of the gene in other individuals.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present invention.

The term "a homologue of a nucleic acid" refers to a nucleic acid having a nucleotide sequence having a certain degree of homology with the nucleotide sequence of the nucleic acid or complement thereof. A homologue of a double stranded nucleic acid having SEQ ID NO. X is intended to include nucleic acids having a nucleotide sequence which has a certain degree of homology with SEQ ID NO. X or with the complement thereof. Preferred homologous of nucleic acids are capable of hybridizing to the nucleic acid or complement thereof.

The term "interact" as used herein is meant to include detectable interactions between molecules, such as can be detected using, for example, a hybridization assay.

The term interact is also meant to include "binding" interactions between molecules. Interactions may be, for example, protein-protein, protein-nucleic acid, protein-small molecule or small molecule-nucleic acid in nature.

The term "intronic sequence" or "intronic nucleotide sequence" refers to the nucleotide sequence of an intron or portion thereof.

The term "isolated" as used herein with respect to nucleic acids, such as DNA or RNA, refers to molecules separated from other DNAs or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized.

Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The term "lipid" shall refer to a fat or fat-like substance that is insoluble in polar solvents such as water. The term "lipid" is intended to include true fats (e.g. esters of fatty acids and glycerol); lipids (phospholipids, cerebrosides, waxes); sterols (cholesterol, ergosterol) and lipoproteins (e.g. HDL, LDL and VLDL).

The term "locus" refers to a specific position in a chromosome. For example, a locus of a gene refers to the chromosomal position of the gene.

The term "modulation" as used herein refers to both upregulation, (i.e., activation or stimulation), for example by agonizing, and downregulation (i.e. inhibition or suppression), for example by antagonizing of a bioactivity (e.g. expression of a gene).

The term "molecular structure" of a gene or a portion thereof refers to the structure as defined by the nucleotide content (including deletions, substitutions, additions of one or more nucleotides), the nucleotide sequence, the state of methylation, and/or any other modification of the gene or portion thereof.

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous (for that gene) subject, the mutation is said to be co-dominant.

As used herein, the term "nucleic acid" refers to polynucleotides such as deoxyribonucleic acid (DNA), and, where appropriate, ribonucleic acid (RNA). The term should also be understood to include, as equivalents, derivatives, variants and analogs of either RNA or DNA made from nucleotide analogs, including peptide nucleic acids (PNA), and, as applicable to the embodiment being described, single (sense or antisense) and double-stranded polynucleotides. Deoxyribonucleotides include deoxyadenosine, deoxycytidine, deoxyguanosine, and deoxythymidine. For purposes of clarity, when referring herein to a nucleotide of a nucleic acid, which can be DNA or an RNA, the term "adenosine", "cytidine", "guanosine", and "thymidine" are used. It is understood that if the nucleic acid is RNA, a nucleotide having a uracil base is uridine.

The term "nucleotide sequence complementary to the nucleotide sequence set forth in SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a non-coding strand. When referring to double stranded nucleic acids, the complement of a nucleic acid having SEQ ID NO. x refers to the complementary strand of the strand having SEQ ID NO. x or to any nucleic acid having the nucleotide sequence of the complementary strand of SEQ ID NO. x. When referring to a single stranded nucleic acid having the nucleotide sequence SEQ ID NO. x, the complement of this nucleic acid is a nucleic acid having a nucleotide sequence which is complementary to that of SEQ ID NO. x. The nucleotide sequences and complementary sequences thereof are always given in the 5' to 3' direction. The term "complement" and "reverse complement" are used interchangeably herein.

The term "operably linked" is intended to mean that the promoter is associated with the nucleic acid in such a manner as to facilitate transcription of the nucleic acid.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

To describe a "polymorphic site" in a nucleotide sequence there is often used an "ambiguity code" that stands for the possible variations of nucleotides in one position. The list of ambiguity codes is summarized in the following table:

| Ambiguity Codes | |
|---|---|
| B | c/g/t |
| D | a/g/t |
| H | a/c/t |
| K | g/t |
| M | a/c |
| N | a/c/g/t |
| R | a/g |
| S | c/g |
| V | a/c/g |
| W | a/t |
| Y | c/t |

So, for example, a "R" in a nucleotide sequence means that either an "a" or a "g" nucleotide could be at that position.

The terms "protein", "polypeptide" and "peptide" are used interchangeably herein when referring to a gene product.

A "regulatory element", also termed herein "regulatory sequence is intended to include elements which are capable of modulating transcription from a basic promoter and include elements such as enhancers and silencers. The term "enhancer", also referred to herein as "enhancer element", is intended to include regulatory elements capable of increasing, stimulating, or enhancing transcription from a basic promoter. The term "silencer", also referred to herein as "silencer element" is intended to include regulatory elements capable of decreasing, inhibiting, or repressing transcription from a basic promoter. Regulatory elements are typically present in 5' flanking regions of genes. However, regulatory elements have also been shown to be present in other regions of a gene, in particular in introns. Thus, it is possible that genes have regulatory elements located in introns, exons, coding regions, and 3' flanking sequences. Such regulatory elements are also intended to be encompassed by the present invention and can be identified by any of the assays that can be used to identify regulatory elements in 5' flanking regions of genes.

The term "regulatory element" further encompasses "tissue specific" regulatory elements, i.e., regulatory elements which effect expression of the selected DNA sequence preferentially in specific cells (e.g., cells of a specific tissue). gene expression occurs preferentially in a specific cell if expression in this cell type is significantly higher than expression in other cell types. The term "regulatory element" also encompasses non-tissue specific regulatory elements, i.e., regulatory elements which are active in most cell types. Furthermore, a regulatory element can be a constitutive regulatory element, i.e., a regulatory element which constitutively regulates transcription, as opposed to a regulatory element which is inducible, i.e., a regulatory element which is active primarily in response to a stimulus. A stimulus can be, e.g., a molecule, such as a hormone, cytokine, heavy metal, phorbol ester, cyclic AMP (cAMP), or retinoic acid.

Regulatory elements are typically bound by proteins, e.g., transcription factors. The term "transcription factor" is intended to include proteins or modified forms thereof, which interact preferentially with specific nucleic acid sequences, i.e., regulatory elements, and which in appropriate conditions stimulate or repress transcription. Some transcription factors are active when they are in the form of a monomer. Alternatively, other transcription factors are active in the form of a dimer consisting of two identical proteins or different proteins (heterodimer). Modified forms of transcription factors are intended to refer to transcription factors having a post-translational modification, such as the attachment of a phosphate group. The activity of a transcription factor is frequently modulated by a post-translational modification. For example, certain transcription factors are active only if they are phosphorylated on specific residues. Alternatively, transcription factors can be active in the absence of phosphorylated residues and become inactivated by phosphorylation. A list of known transcription factors and their DNA binding site can be found, e.g., in public databases, e.g., TFMATRIX Transcription Factor Binding Site Profile database.

As used herein, the term "specifically hybridizes" or "specifically detects" refers to the ability of a nucleic acid molecule of the invention to hybridize to at least approximately 6, 12, 20, 30, 40, 50, 60, 70, 80, 90, 100, 110, 120, 130 or 140 consecutive nucleotides of either strand of a gene.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

### Methods for Assessing Cardiovascular Status

The present invention provides diagnostic methods for assessing cardiovascular status in a human individual. Cardiovascular status as used herein refers to the physiological status of an individual's cardiovascular system as reflected in one or more markers or indicators. Status markers include without limitation clinical measurements such as, e.g., blood pressure, electrocardiographic profile, and differentiated blood flow analysis as well as measurements of LDL- and HDL-Cholesterol levels, other lipids and other well established clinical parameters that are standard in the art. Status markers according to the invention include diagnoses of one or more cardiovascular syndromes, such as, e.g., hypertension, acute myocardial infarction, silent myocardial infarction, stroke, and atherosclerosis. It will be understood that a diagnosis of a cardiovascular syndrome made by a medical practitioner encompasses clinical measurements and medical judgement. Status markers according to the invention are assessed using conventional methods well known in the art. Also included in the evaluation of cardiovascular status are quantitative or qualitative changes in status markers with time, such as would be used, e.g., in the determination of an individual's response to a particular therapeutic regimen.

The methods are carried out by the steps of:
(i) determining the sequence of one or more polymorphic positions within one or more of the genes encoding Na⁺/K⁺ ATPases or other genes mentioned in this file in the individual to establish a polymorphic pattern for the individual; and
(ii) comparing the polymorphic pattern established in (i) with the polymorphic patterns of humans exhibiting different markers of cardiovascular status. The polymorphic pattern of the individual is, preferably, highly similar and, most preferably, identical to the polymorphic pattern of individuals who exhibit particular status markers, cardiovascular syndromes, and/or particular patterns of response to therapeutic interventions. Polymorphic patterns may also include polymorphic positions in other genes which are shown, in combination with one or more polymorphic positions in Na⁺/K⁺ ATPase, to correlate with the presence of particular status markers. In one embodiment, the method involves comparing an individual's polymorphic pattern with polymorphic patterns of individuals who have been shown to respond positively or negatively to a particular therapeutic regimen. Therapeutic regimen as used herein refers to treatments aimed at the elimination or amelioration of symptoms and events associated cardiovascular disease. Such treatments include without limitation one or more of alteration in diet, lifestyle, and exercise regimen; invasive and noninvasive surgical techniques such as atherectomy, angioplasty, and coronary bypass surgery; and pharmaceutical interventions, such as administration of ACE inhibitors, angiotensin II receptor antagonists, diuretics, alpha-adrenoreceptor antagonists, cardiac glycosides, phosphodiesterase inhibitors, beta-adrenoreceptor antagonists, calcium channel blockers, HMG-CoA reductase inhibitors, imidazoline receptor blockers, endothelin receptor blockers, organic nitrites, and Na⁺/K⁺ ATPase modulators. Interventions with pharmaceutical agents not yet known whose activity correlates with particular polymorphic patterns associated with cardiovascular disease are also encompassed. It is contemplated, for example, that patients who are candidates for a particular therapeutic regimen will be screened for polymorphic patterns that correlate with responsivity to that particular regimen.

In a preferred embodiment, the method involves comparing an individual's polymorphic pattern with polymorphic patterns of individuals who exhibit or have exhibited one or more markers of cardiovascular disease, such as, e.g., elevated LDL-Cholesterol levels, high blood pressure, abnormal electrocardiographic profile, myocardial infarction, stroke, or atherosclerosis.

In practicing the methods of the invention, an individual's polymorphic pattern can be established by obtaining DNA from the individual and determining the sequence at predetermined polymorphic positions in Na⁺/K⁺ ATPase and other genes such as those described in this file.

The DNA may be obtained from any cell source. Non-limiting examples of cell sources available in clinical practice include blood cells, buccal cells, cervicovaginal cells, epithelial cells from urine, fetal cells, or any cells present in tissue obtained by biopsy. Cells may also be obtained from body fluids, including without limitation blood, saliva, sweat, urine, cerebrospinal fluid, feces, and tissue exudates at the site of infection or inflammation. DNA is extracted from the cell source or body fluid using any of the numerous methods that are standard in the art. It will be understood that the particular method used to extract DNA will depend on the nature of the source.

### Diagnostic and Prognostic Assays

The present invention provides methods for determining the molecular structure of at least one polymorphic region of a gene, specific allelic variants of said polymorphic region being associated with cardiovascular disease. In one embodiment, determining the molecular structure of a polymorphic region of a gene comprises determining the identity of the allelic variant. A polymorphic region of a gene, of which specific alleles are associated with cardiovascular disease can be located in an exon, an intron, at an intron/exon border, or in the promoter of the gene.

The invention provides methods for determining whether a subject has, or is at risk, of developing a cardiovascular disease. Such disorders can be associated with an aberrant gene activity, e.g., abnormal binding to a form of a lipid, or an aberrant gene protein level. An aberrant gene protein level can result from an aberrant transcription or post-transcriptional regulation. Thus, allelic differences in specific regions of a gene can result in differences of gene protein due to differences in regulation of expression. In particular, some of the identified polymorphisms in the human gene may be associated with differences in the level of transcription, RNA maturation, splicing, or translation of the gene or transcription product.

In preferred embodiments, the methods of the invention can be characterized as comprising detecting, in a sample of cells from the subject, the presence or absence of a specific allelic variant of one or more polymorphic regions of a gene. The allelic differences can be: (i) a difference in the identity of at least one nucleotide or (ii) a difference in the number of nucleotides, which difference can be a single nucleotide or several nucleotides.

A preferred detection method is allele specific hybridization using probes overlapping the polymorphic site and having about 5, 10, 20, 25, or 30 nucleotides around the polymorphic region. Examples of probes for detecting specific allelic variants of the polymorphic region located in intron X are probes comprising a nucleotide sequence set forth in any of SEQ ID NO. X. In a preferred embodiment of the invention, several probes capable of hybridizing specifically to allelic variants are attached to a solid phase support, e.g., a "chip". Oligonucleotides can be bound to a solid support by a variety of processes, including lithography. For example a chip can hold up to 250,000 oligonucleotides (GeneChip, Affymetrix). Mutation detection analysis using these chips comprising oligonucleotides, also termed "DNA probe arrays" is described e.g., in Cronin et al. (1996) Human Mutation 7:244 and in Kozal et al. (1996) Nature Medicine 2:753. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment. For example, the identity of the allelic variant of the nucleotide polymorphism of nucleotide A or G at position 240 of Seq ID (baySNP1765) and that of other possible polymorphic regions can be determined in a single hybridization experiment.

In other detection methods, it is necessary to first amplify at least a portion of a gene prior to identifying the allelic variant. Amplification can be performed, e.g., by PCR and/or LCR, according to methods known in the art. In one embodiment, genomic DNA of a cell is exposed to two PCR primers and amplification for a number of cycles sufficient to produce the required amount of amplified DNA. In preferred embodiments, the primers are located between 150 and 350 base pairs apart. Preferred primers, such as primers for amplifying of an intron of the human Na⁺/K⁺ ATPase gene, are listed in Table 2 in the Examples.

Alternative amplification methods include: self sustained sequence replication (Guatelli, J. C. et al., 1990, Proc. Natl. Acad. Sci. U.S.A. 87:1874-1878), transcriptional amplification system (Kwoh, D. Y. et al., 1989, Proc. Natl. Acad. Sci. U.S.A. 86:1173-1177), Q-Beta Replicase (Lizardi, P. M. et al., 1988, Bio/-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In one embodiment, any of a variety of sequencing reactions known in the art can be used to directly sequence at least a portion of a gene and detect allelic variants, e.g., mutations, by comparing the sequence of the sample sequence with the corresponding wild-type (control) sequence. Exemplary sequencing reactions include those based on techniques developed by Maxam and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (Biotechniques (1995) 19:448), including sequencing by mass spectrometry (see, for example, U.S. Pat. No. 5,547,835 and international patent application Publication Number WO 94/16101, entitled DNA Sequencing by Mass Spectrometry by H. Koster; U.S. Pat. No. 5,547,835 and international patent application Publication Number WO 94/21822 entitled "DNA Sequencing by Mass Spectrometry Via Exonuclease Degradation" by H. Koster), and U.S. Pat. No. 5,605,798 and International Patent Application No. PCT/US96/03651 entitled DNA Diagnostics Based on Mass Spectrometry by H. Koster; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-track or the like, e.g., where only one nucleotide is detected, can be carried out.

Yet other sequencing methods are disclosed, e.g., in U.S. Pat. No. 5,580,732 entitled "Method of DNA sequencing employing a mixed DNA-polymer chain probe" and U.S. Pat. No. 5,571,676 entitled "Method for mismatch-directed in vitro DNA sequencing".

In some cases, the presence of a specific allele of a gene in DNA from a subject can be shown by restriction enzyme analysis. For example, a specific nucleotide polymorphism can result in a nucleotide sequence comprising a restriction site which is absent from the nucleotide sequence of another allelic variant.

In other embodiments, alterations in electrophoretic mobility is used to identify the type of gene allelic variant. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control nucleic acids are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In another preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the identity of an allelic variant of a polymorphic region is obtained by analyzing the movement of a nucleic acid comprising the polymorphic region in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:1275).

Examples of techniques for detecting differences of at least one nucleotide between 2 nucleic acids include, but are not limited to, selective oligonucleotide hybridization, selective amplification, or selective primer extension. For example, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific allelic variants are attached to a hybridizing membrane and this membrane is then hybridized with labeled sample nucleic acid. Analysis of the hybridization signal will then reveal the identity of the nucleotides of the sample nucleic acid.

Alternatively, allele specific amplification technology which depends on selective PCR amplification may be used. Oligonucleotides used as primers for specific amplification may carry the allelic variant of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238; Newton et al. (1989) Nucl. Acids Res. 17:2503). This technique is also termed "PROBE" for Probe Oligo Base Extension. In addition it may be desirable to introduce a novel restriction site in the region of the mutation to create cleavage-based detection (Gasparini et al (1992) Mol. Cell Probes 6:1).

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA, which is then detected using OLA.

Several techniques based on this OLA method have been developed and can be used to detect specific allelic variants of a polymorphic region of a gene. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996)Nucleic Acids Res 24: 3728), OLA combined with PCR permits typing of two alleles in a single microtiter well. By marking each of the allele-specific primers with a unique hapten, i.e. digoxigenin and fluorescein, each LA reaction can be detected by using hapten specific antibodies that are labeled with different enzyme reporters, alkaline phosphatase or horseradish peroxidase. This system permits the detection of the two alleles using a high throughput format that leads to the production of two different colors.

The invention further provides methods for detecting single nucleotide polymorphisms in a gene. Because single nucleotide polymorphisms constitute sites of variation flanked by regions of invariant sequence, their analysis requires no more than the determination of the identity of the single nucleotide present at the site of variation and it is unnecessary to determine a complete gene sequence for each patient. Several methods have been developed to facilitate the analysis of such single nucleotide polymorphisms.

In one embodiment, the single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA TM is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990), Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA TM in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For determining the identity of the allelic variant of a polymorphic region located in the coding region of a gene, yet other methods than those described above can be used. For example, identification of an allelic variant which encodes a mutated gene protein can be performed by using an antibody specifically recognizing the mutant protein in, e.g., immunohistochemistry or immunoprecipitation. Antibodies to wild-type gene protein are described, e.g., in Acton et al. (1999) Science 271:518 (antimouse gene antibody cross-reactive with human gene). Other antibodies to wild-type gene or mutated forms of gene proteins can be prepared according to methods known in the art. Alternatively, one can also measure an activity of an gene protein, such as binding to a lipid or lipoprotein. Binding assays are known in the art and involve, e.g., obtaining cells from a subject, and performing binding experiments with a labeled lipid, to determine whether binding to the mutated form of the receptor differs from binding to the wild-type of the receptor.

If a polymorphic region is located in an exon, either in a coding or non-coding region of the gene, the identity of the allelic variant can be determined by determining the molecular structure of the mRNA, pre-mRNA, or cDNA. The molecular structure can be determined using any of the above described methods for determining the molecular structure of the genomic DNA, e.g., sequencing and SSCP.

The methods described herein may be performed, for example, by utilizing prepackaged diagnostic kits, such as those described above, comprising at least one probe or primer nucleic acid described herein, which may be conveniently used, e.g., to determine whether a subject has or is at risk of developing a disease associated with a specific gene allelic variant.

Sample nucleic acid for using in the above-described diagnostic and prognostic methods can be obtained from any cell type or tissue of a subject. For example, a subject's bodily fluid (e.g. blood) can be obtained by known techniques (e.g. venipuncture) or from human tissues like heart (biopsies, transplanted organs). Alternatively, nucleic acid tests can be performed on dry samples (e.g. hair or skin). Fetal nucleic acid samples for prenatal diagnostics can be obtained from maternal blood as described in International Patent Application No.WO91/07660 to Bianchi.

Alternatively, amniocytes or chorionic villi may be obtained for performing prenatal testing.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, New York).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

In practicing the present invention, the distribution of polymorphic patterns in a large number of individuals exhibiting particular markers of cardiovascular status is determined by any of the methods described above, and compared with the distribution of polymorphic patterns in patients that have been matched for age, ethnic origin, and/or any other statistically or medically relevant parameters, who exhibit quantitatively or qualitatively different status markers. Correlations are achieved using any method known in the art, including nominal logistic regression, chi square tests or standard least squares regression analysis. In this manner, it is possible to establish statistically significant correlations between particular polymorphic patterns and particular cardiovascular statuses (given in p values). It is further possible to establish statistically significant correlations between particular polymorphic patterns and changes in cardiovascular status such as, would result, e.g., from particular treatment regimens. In this manner, it is possible to correlate polymorphic patterns with responsivity to particular treatments.

### Isolated Polymorphic Nucleic Acids, Probes, and Vectors

The present invention provides isolated nucleic acids comprising the polymorphic positions described herein for the human Na⁺/K⁺ ATPase, peroxisomal farnesylated protein (PXF) or coatomer protein complex (COPA) genes; vectors comprising the nucleic acids; and transformed host cells comprising the vectors. The invention also provides probes which are useful for detecting these polymorphisms.

In practicing the present invention, many conventional techniques in molecular biology, microbiology, and recombinant DNA, are used. Such techniques are well known and are explained fully in, for example, Sambrook et al., 1989, Molecular Cloning: A Laboratory Manual, Second Edition, Cold Spring Harbor Laboratory Press, Cold Spring Harbor, New York; DNA Cloning: A Practical Approach, Volumes I and II, 1985 (D. N. Glover ed.); Oligonucleotide Synthesis, 1984, (M. L.Gait ed.); Nucleic Acid Hybridization, 1985, (Hames and Higgins); Ausubel et al., Current Protocols in Molecular Biology, 1997, (John Wiley and Sons); and Methods in Enzymology Vol. 154 and Vol. 155 (Wu and Grossman, and Wu, eds., respectively).

Insertion of nucleic acids (typically DNAs) comprising the sequences in a functional surrounding like full length cDNA of the present invention into a vector is easily accomplished when the termini of both the DNAs and the vector comprise compatible restriction sites. If this cannot be done, it may be necessary to modify the termini of the DNAs and/or vector by digesting back single-stranded DNA overhangs generated by restriction endonuclease cleavage to produce blunt ends, or to achieve the same result by filling in the single-stranded termini with an appropriate DNA polymerase.

Alternatively, any site desired may be produced, e.g., by ligating nucleotide sequences (linkers) onto the termini. Such linkers may comprise specific oligonucleotide sequences that define desired restriction sites. Restriction sites can also be generated by the use of the polymerase chain reaction (PCR). See, e.g., Saiki et al., 1988, Science 239:48. The cleaved vector and the DNA fragments may also be modified if required by homopolymeric tailing.

The nucleic acids may be isolated directly from cells or may be chemically synthesized using known methods. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression.

The nucleic acids of the present invention may be flanked by native Na⁺/K⁺ ATPase, PXF, or COPA gene sequences, respectively, or may be associated with heterologous sequences, including promoters, enhancers, response elements, signal sequences, polyadenylation sequences, introns, 5'- and 3'-noncoding regions, and the like. The nucleic acids may also be modified by many means known in the art. Non-limiting examples of such modifications include methylation, "caps", substitution of one or more of the naturally occurring nucleotides with an analog, internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. PNAs are also included. The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

The invention also provides nucleic acid vectors comprising the Na⁺/K⁺ ATPase-, PXF-, or COPA-derived gene sequences or derivatives or fragments thereof. A large number of vectors, including plasmid and fungal vectors, have been described for replication and/or expression in a variety of eukaryotic and prokaryotic hosts, and may be used for gene therapy as well as for simple cloning or protein expression. Non-limiting examples of suitable vectors include without limitation pUC plasmids, pET plasmids (Novagen, Inc., Madison, Wis.), or pRSET or pREP (Invitrogen, San Diego, Calif.), and many appropriate host cells, using methods disclosed or cited herein or otherwise known to those skilled in the relevant art. The particular choice of vector/host is not critical to the practice of the invention.

Suitable host cells may be transformed/transfected/infected as appropriate by any suitable method including electroporation, CaCl₂ mediated DNA uptake, fungal or viral infection, microinjection, microprojectile, or other established methods. Appropriate host cells included bacteria, archebacteria, fungi, especially yeast, and plant and animal cells, especially mammalian cells. A large number of transcription initiation and termination regulatory regions have been isolated and shown to be effective in the transcription and translation of heterologous proteins in the various hosts. Examples of these regions, methods of isolation, manner of manipulation, etc. are known in the art. Under appropriate expression conditions, host cells can be used as a source of recombinantly produced Na⁺/K⁺ ATPase-, PXF-, or COPA-derived peptides and polypeptides. Nucleic acids encoding Na⁺/K⁺ ATPase-, PXF-, or COPA-derived gene sequences may also be introduced into cells by recombination events. For example, such a sequence can be introduced into a cell and thereby effect homologous recombination at the site of an endogenous gene or a sequence with substantial identity to the gene. Other recombination-based methods such as nonhomologous recombinations or deletion of endogenous genes by homologous recombination may also be used.

In case of proteins that form heterodimers or other multimers, like the Na⁺/K⁺ ATPases both or all subunits have to be expressed in one system or cell. A list of those can be found in Table 4.

The nucleic acids of the present invention find use as probes for the detection of genetic polymorphisms and as templates for the recombinant production of normal or variant Na⁺/K⁺ ATPase-, PXF-, or COPA-derived peptides or polypeptides.

Probes in accordance with the present invention comprise without limitation isolated nucleic acids of about 10-100 bp, preferably 15-75 bp and most preferably 17-25 bp in length, which hybridize at high stringency to one or more of the Na⁺/K⁺ ATPase, PXF, or COPA gene-derived polymorphic sequences disclosed herein or to a sequence immediately adjacent to a polymorphic position. Furthermore, in some embodiments a full-length gene sequence may be used as a probe. In one series of embodiments, the probes span the polymorphic positions in the Na⁺/K⁺ ATPase, PXF, or COPA genes disclosed herein. In another series of embodiments, the probes correspond to sequences immediately adjacent to the polymorphic positions.

### Polymorphic Na⁺/K⁺ ATPase, PXF, or COPA Polypeptides and Polymorphism-Specific Antibodies

The present invention encompasses isolated peptides and polypeptides encoding Na⁺/K⁺ ATPase, PXF, or COPA comprising polymorphic positions disclosed herein. In one preferred embodiment, the peptides and polypeptides are useful screening targets to identify cardiovascular drugs. In another preferred embodiments, the peptides and polypeptides are capable of eliciting antibodies in a suitable host animal that react specifically with a polypeptide comprising the polymorphic position and distinguish it from other polypeptides having a different sequence at that position.

Polypeptides according to the invention are preferably at least five or more residues in length, preferably at least fifteen residues. Methods for obtaining these poly-peptides are described below. Many conventional techniques in protein biochemistry and immunology are used. Such techniques are well known and are explained in Immunochemical Methods in Cell and Molecular Biology, 1987 (Mayer and Waler, eds; Academic Press, London); Scopes, 1987, Protein Purification: Principles and Practice, Second Edition (Springer-Verlag, N.Y.) and Handbook of Experimental Immunology, 1986, Volumes I-IV (Weir and Blackwell eds.).

Nucleic acids comprising protein-coding sequences can be used to direct the ITT recombinant expression of Na⁺/K⁺ ATPase-, PXF-, or COPA-derived polypeptides in intact cells or in cell-free translation systems. The known genetic code, tailored if desired for more efficient expression in a given host organism, can be used to synthesize oligonucleotides encoding the desired amino acid sequences. The polypeptides may be isolated from human cells, or from heterologous organisms or cells (including, but not limited to, bacteria, fungi, insect, plant, and mammalian cells) into which an appropriate protein-coding sequence has been introduced and expressed. Furthermore, the polypeptides may be part of recombinant fusion proteins.

Peptides and polypeptides may be chemically synthesized by commercially available automated procedures, including, without limitation, exclusive solid phase synthesis, partial solid phase methods, fragment condensation or classical solution synthesis. The polypeptides are preferably prepared by solid phase peptide synthesis as described by Merrifield, 1963, J. Am. Chem. Soc. 85:2149.

Methods for polypeptide purification are well-known in the art, including, without limitation, preparative disc-gel electrophoresis, isoelectric focusing, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against Na⁺/K⁺ ATPase, PXF, or COPA, or against peptides derived from those, can be used as purification reagents. Other purification methods are possible.

The present invention also encompasses derivatives and homologues of the polypeptides. For some purposes, nucleic acid sequences encoding the peptides may be altered by substitutions, additions, or deletions that provide for functionally equivalent molecules, i.e., function-conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non-polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

The present invention also encompasses antibodies that specifically recognize the polymorphic positions of the invention and distinguish a peptide or polypeptide containing a particular polymorphism from one that contains a different sequence at that position. Such polymorphic position-specific antibodies according to the present invention include polyclonal and monoclonal antibodies. The antibodies may be elicited in an animal host by immunization with Na⁺/K⁺ ATPase-, PXF-, or COPA-derived immunogenic components or may be formed by in vitro immunization of immune cells. The immunogenic components used to elicit the antibodies may be isolated from human cells or produced in recombinant systems. The antibodies may also be produced in recombinant systems programmed with appropriate antibody-encoding DNA. Alternatively, the antibodies may be constructed by biochemical reconstitution of purified heavy and light chains. The antibodies include hybrid antibodies (i.e., containing two sets of heavy chain/light chain combinations, each of which recognizes a different antigen), chimeric antibodies (i.e., in which either the heavy chains, light chains, or both, are fusion proteins), and univalent antibodies (i.e., comprised of a heavy chain/light chain complex bound to the constant region of a second heavy chain). Also included are Fab fragments, including Fab' and F(ab).sub.2 fragments of antibodies. Methods for the production of all of the above types of antibodies and derivatives are well-known in the art and are discussed in more detail below. For example, techniques for producing and processing polyclonal antisera are disclosed in Mayer and Walker, 1987, Immunochemical Methods in Cell and Molecular Biology, (Academic Press, London). The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., Schreier et al., 1980, Hybridoma Techniques; U.S. Pat. Nos. 4,341,761; 4,399,121; 4,427,783; 4,444,887; 4,466,917; 4,472,500; 4,491,632; and 4,493,890. Panels of monoclonal antibodies produced against Na⁺/K⁺ ATPase-, PXF-, or COPA-derived epitopes can be screened for various properties; i.e. for isotype, epitope affinity, etc.

The antibodies of this invention can be purified by standard methods, including but not limited to preparative disc-gel electrophoresis, isoelectric focusing, HPLC, reversed-phase HPLC, gel filtration, ion exchange and partition chromatography, and countercurrent distribution. Purification methods for antibodies are disclosed, e.g., in The Art of Antibody Purification, 1989, Amicon Division, W. R. Grace & Co. General protein purification methods are described in Protein Purification: Principles and Practice, R. K. Scopes, Ed., 1987, Springer-Verlag, New York, N.Y.

Methods for determining the immunogenic capability of the disclosed sequences and the characteristics of the resulting sequence-specific antibodies and immune cells are well-known in the art. For example, antibodies elicited in response to a peptide comprising a particular polymorphic sequence can be tested for their ability to specifically recognize that polymorphic sequence, i.e., to bind differentially to a peptide or polypeptide comprising the polymorphic sequence and thus distinguish it from a similar peptide or polypeptide containing a different sequence at the same position.

### Kits

As set forth herein, the invention provides diagnostic methods, e.g., for determining the identity of the allelic variant of a polymorphic region present in the Na⁺/K⁺ ATPase gene locus, wherein specific allelic variants of the polymorphic region are associated with cardiovascular diseases. In a preferred embodiment, the diagnostic kit can be used to determine whether a subject is at risk of developing a cardiovascular disease. This information could then be used, e.g., to optimize treatment of such individuals.

In preferred embodiments, the kit comprises a probe or primer which is capable of hybridizing to a gene and thereby identifying whether the gene contains an allelic variant of a polymorphic region which is associated with a risk for cardiovascular disease. The kit preferably further comprises instructions for use in diagnosing a subject as having, or having a predisposition, towards developing a cardiovascular disease. The probe or primers of the kit can be any of the probes or primers described in this file.

Preferred kits for amplifying a region of a gene comprising a polymorphic region of interest comprise one, two or more primers.

### Antibody-based diagnostic methods and kits

The invention also provides antibody-based methods for detecting polymorphic patterns in a biological sample. The methods comprise the steps of: (i) contacting a sample with one or more antibody preparations, wherein each of the antibody preparations is specific for a particular polymorphic form of Na⁺/K⁺ ATPases, PXF, or COPA, under conditions in which a stable antigen-antibody complex can form between the antibody and antigenic components in the sample; and (ii) detecting any antigen-antibody complex formed in step (i) using any suitable means known in the art, wherein the detection of a complex indicates the presence of the particular polymorphic form in the sample.

Typically, immunoassays use either a labelled antibody or a labelled antigenic component (e.g., that competes with the antigen in the sample for binding to the antibody). Suitable labels include without limitation enzyme-based, fluorescent, chemiluminescent, radioactive, or dye molecules. Assays that amplify the signals from the probe are also known, such as, for example, those that utilize biotin and avidin, and enzyme-labelled immunoassays, such as ELISA assays.

The present invention also provides kits suitable for antibody-based diagnostic applications. Diagnostic kits typically include one or more of the following components:
(i) Polymorphism-specific antibodies. The antibodies may be pre-labelled; alternatively, the antibody may be unlabelled and the ingredients for labelling may be included in the kit in separate containers, or a secondary, labelled antibody is provided; and
(ii) Reaction components: The kit may also contain other suitably packaged reagents and materials needed for the particular immunoassay protocol, including solid-phase matrices, if applicable, and standards.

The kits referred to above may include instructions for conducting the test. Furthermore, in preferred embodiments, the diagnostic kits are adaptable to high-throughput and/or automated operation.

### Drug Targets and Screening Methods

According to the present invention, nucleotide sequences derived from genes encoding Na⁺/K⁺ ATPase, PXF, or COPA and peptide sequences derived from Na⁺/K⁺ ATPase, PXF, or COPA polypeptides, particularly those that contain one or more polymorphic sequences, comprise useful targets to identify cardiovascular drugs, i.e., compounds that are effective in treating one or more clinical symptoms of cardiovascular disease. Furthermore, especially when a protein is a multimeric protein, like the Na⁺/K⁺ ATPases that are build of α- and β subunits, is a combination of different polymorphic α subunits to different polymorphic β subunits very useful.

Drug targets include without limitation (i) isolated nucleic acids derived from the genes encoding Na⁺/K⁺ ATPase, PXF, or COPA, and (ii) isolated peptides and polypeptides derived from Na⁺/K⁺ ATPase, PXF, or COPA polypeptides, each of which comprises one or more polymorphic positions.

### In vitro screening methods

In one series of embodiments, an isolated nucleic acid comprising one or more polymorphic positions is tested in vitro for its ability to bind test compounds in a sequence-specific manner. The methods comprise:
(i) providing a first nucleic acid containing a particular sequence at a polymorphic position and a second nucleic acid whose sequence is identical to that of the first nucleic acid except for a different sequence at the same polymorphic position;
(ii) contacting the nucleic acids with a multiplicity of test compounds under conditions appropriate for binding; and
(iii) identifying those compounds that bind selectively to either the first or second nucleic acid sequence.

Selective binding as used herein refers to any measurable difference in any parameter of binding, such as, e.g., binding affinity, binding capacity, etc.

In another series of embodiments, an isolated peptide or polypeptide comprising one or more polymorphic positions is tested in vitro for its ability to bind test compounds in a sequence-specific manner. The screening methods involve:
(i) providing a first peptide or polypeptide containing a particular sequence at a polymorphic position and a second peptide or polypeptide whose sequence is identical to the first peptide or polypeptide except for a different sequence at the same polymorphic position;
(ii) contacting the polypeptides with a multiplicity of test compounds under conditions appropriate for binding; and
(iii) identifying those compounds that bind selectively to one of the nucleic acid sequences.

In preferred embodiments, high-throughput screening protocols are used to survey a large number of test compounds for their ability to bind the genes or peptides disclosed above in a sequence-specific manner.

Test compounds are screened from large libraries of synthetic or natural compounds. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means.

### In vivo screening methods

Intact cells or whole animals expressing polymorphic variants of genes encoding Na⁺/K⁺ ATPases, PXF, or COPA can be used in screening methods to identify candidate cardiovascular drugs.

In one series of embodiments, a permanent cell line is established from an individual exhibiting a particular polymorphic pattern. Alternatively, cells (including without limitation mammalian, insect, yeast, or bacterial cells) are programmed to express a gene comprising one or more polymorphic sequences by introduction of appropriate DNA. Identification of candidate compounds can be achieved using any suitable assay, including without limitation (i) assays that measure selective binding of test compounds to particular polymorphic variants of Na⁺/K⁺ ATPase; (ii) assays that measure the ability of a test compound to modify (i.e., inhibit or enhance) a measurable activity or function of Na⁺/K⁺ ATPase, PXF, or COPA; and (iii) assays that measure the ability of a compound to modify (i.e., inhibit or enhance) the transcriptional activity of sequences derived from the promoter (i.e., regulatory) regions of Na⁺/K⁺ ATPase, PXF, or COPA genes.

In another series of embodiments, transgenic animals are created in which (i) one or more human Na⁺/K⁺ ATPase, PXF, or COPA genes, respectively, having different sequences at particular polymorphic positions are stably inserted into the genome of the transgenic animal; and/or (ii) the endogenous Na⁺/K⁺ ATPase, PXF, or COPA genes are inactivated and replaced with human Na⁺/K⁺ ATPase, PXF, or COPA genes, respectively, having different sequences at particular polymorphic positions.

See, e.g., Coffman, Semin. Nephrol. 17:404, 1997; Esther et al., Lab. Invest. 74:953, 1996; Murakami et al., Blood Press. Suppl. 2:36, 1996. Such animals can be treated with candidate compounds and monitored for one or more clinical markers of cardiovascular status.

The following are intended as non-limiting examples of the invention.

### Material and Methods

Genotyping of patient DNA with the Pyrosequencing™ Method as described in the patent application WO 9813523:

First a PCR is set up to amplify the flanking regions around a SNP. Therefor 2 ng of genomic DNA (patient sample) are mixed with a primerset (20 - 40 pmol) producing a 75 to 320 bp PCR fragment with 0,3 to 1 U Qiagens Hot Star Taq Polymerase™ in a total volume of 20 µL. One primer is biotinylated depending on the direction of the sequencing primer. To force the biotinylated primer to be incorporated it is used 0,8 fold.

For primer design, programms like Oligo 6™ (Molecular Biology Insights) or Primer Select™ (DNAStar) are used. PCR setup is performed by a BioRobot 3000 ™ from Qiagen. PCR takes place in T1 or Tgradient Thermocyclers™ from Biometra.

The whole PCR reaction is transferred into a PSQ plate ™ (Pyrosequencing) and prepared using the Sample Prep Tool ™ and SNP Reagent Kit ™ from Pyrosequencing according to their instructions.

### Preparation of template for Pyrosequencing™:

Sample preparation using PSQ 96 Sample Prep Tool
1. Mount the PSQ 96 Sample Prep Tool Cover onto the PSQ 96 Sample Prep Tool as follows: Place the cover on the desk, retract the 4 attachment rods by separating the handle from the magnetic rod holder, fit the magnetic rods into the holes of the cover plate, push the handle downward until a click is heard. The PSQ 96 Sample Prep Tool is now ready for use.
2. To transfer beads from one plate to another, place the covered tool into the PSQ 96 Plate containing the samples and lower the magnetic rods by separating the handle from the magnetic rod holder. Move the tool up and down a few times then wait for 30-60 seconds. Transfer the beads into a new PSQ 96 plate containing the solution of choice.
3. Release the beads by lifting the magnetic rod holder, bringing it together with the handle. Move the tool up and down a few times to make sure that the beads are released.

All steps are performed at room temperature unless otherwise stated.

### Immobilization of PCR product:

Biotinylated PCR products are immobilized on streptavidin-coated Dynabeads™ M-280 Streptavidin. Parallel immobilization of several samples are performed in the PSQ 96 Plate.
1. Mix PCR product, 20 µl of a well optimized PCR, with 25 µl 2X BW-buffer II. Add 60-150 µg Dynabeads. It is also possible to add a mix of Dynabeads and 2X BW-buffer II to the PCR product yielding a final BW-buffer II concentration of approximately 1x.
2. Incubate at 65°C for 15 min agitation constantly to keep the beads dispersed. For optimal immobilization of fragments longer than 300 bp use 30 min incubation time.

### Strand separation

4. For strand separation, use the PSQ 96 Sample Prep Tool to transfer the beads with the immobilized sample to a PSQ 96 Plate containing 50 µl 0.50 M NaOH per well. Release the beads.
5. After approximately 1 min, transfer the beads with the immobilized strand to a PSQ 96 Plate containing 99 µl 1x Annealing buffer per well and mix thoroughly.
6. Transfer the beads to a PSQ 96 Plate containing 45 µl of a mix of 1x Annealing buffer and 3-15 pmoles sequencing primer per well.
7. Heat at 80°C for 2 minutes in the PSQ 96 Sample Prep Thermoplate and move to room temperature.
8. After reaching room temperature, continue with the sequencing reaction.

### Sequencing reaction

1. Choose the method to be used ("SNP Method") and enter relevant information in the PSQ 96 Instrument Control software.
2. Place the cartridge and PSQ 96 Plate in the PSQ 96 Instrument.
3. Start the run.

### Genotyping with a service contractor

Qiagen Genomics, formerly Rapigene, is a service contractor for genotyping SNPs in patient samples. Their method is based on a primer extension method where two complementary primers are designed for each genotype that are labeled with different tags. Depending on the genotype only one primer will be elongated together with a certain tag. This tag can be detected with mass spectrometry and is a measure for the respective genotype. The method is described in the following patent: "Detection and identification of nucleic acid molecules - using tags which may be detected by non-fluorescent spectrometry or potentiometry" (WO 9727325).

### Examples

### Example 1

Patient cohorts: 107 patients suffering from myocardial infarction as examined by a cardiologist and 75 healthy controls matched in age and sex and without any signs of cardiovascular risk.

### Example 2

Patient cohorts: 278 patient with so called "good" and "bad" serum lipid levels as defined in Table 1 and ages between 65 and 80 years were examined:

**Table 1:**

| Definition of "good" and "bad" serum lipid levels | | |
|---|---|---|
| | "Good" | "Bad" |
| LDL-Cholesterol [mg/dL] | 125 -150 | 170 - 200 |
| Cholesterol [mg/dL] | 190 - 240 | 265 - 315 |
| HDL-Cholesterol [mg/dL] | 60 -105 | 30 - 55 |
| Triglycerides [mg/dL] | 45 - 115 | 170 - 450 |
| Number of Patients | 146 | 132 |

An informed consent was signed by the patients and control people. Blood was taken by a physician according to medical standard procedures.

Samples were collected anonymous and labeled with a patient number.

DNA was extracted using kits from Qiagen.

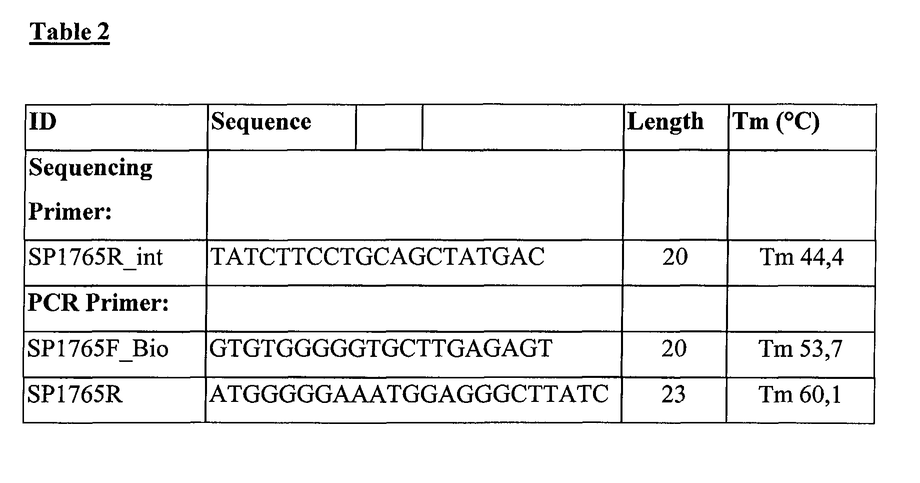

**Table 3**

| baySNP-1765 | | | | | |
|---|---|---|---|---|---|
| Human Na,K-ATPase subunit alpha 2 (ATP1A2) gene | | | | | |
| GTYPE11 | GTYPE12 | GTYPE22 | FREQ1 | FREQ2 | |
| AA | AG | GG | 168 | 810 | |

| FREQ11 | FREQ12 | FREQ22 | %FREQ1 | %FREQ2 | |
|---|---|---|---|---|---|
| 21 | 126 | 342 | 17 | 83 | |
| | | | | | |

| COHORT A | SIZE A | HW PVALUE A | | | |
|---|---|---|---|---|---|
| ALL BAD | 104 | 0,0099 | | | |
| FEMALE BAD | 85 | 0,0382 | | | |
| | | | | | |

| | FREQ1 A | FREQ2 A | FREQ11 A | FREQ12A | FREQ22 A |
|---|---|---|---|---|---|
| ALL BAD | 34 | 174 | 6 | 22 | 76 |
| FEMALE BAD | 25 | 145 | 4 | 17 | 64 |
| | | | | | |

| | %FREQ1 A | %FREQ2 A | %FREQ11 A | %FREQ12 A | %FREQ22 A |
|---|---|---|---|---|---|
| ALL BAD | 16 | 84 | 6 | 21 | 73 |
| FEMALE BAD | 15 | 85 | 5 | 20 | 75 |
| | | | | | |
| | | | | | |

| COHORT B | SIZE B | HW PVALUE B | | | |
|---|---|---|---|---|---|
| ALL GOOD | 123 | 0,2293 | | | |
| FEMALE GOOD | 82 | 0,2454 | | | |
| | | | | | |

| | FREQ1 B | FREQ2 B | FREQ11B | FREQ12B | FREQ22 B |
|---|---|---|---|---|---|
| ALL GOOD | 49 | 197 | 3 | 43 | 77 |
| FEMALE GOOD | 34 | 130 | 2 | 30 | 50 |
| | | | | | |

| | %FREQ1 B | %FREQ2 B | %FREQ11 B | %FREQ12 B | %FREQ22 B |
|---|---|---|---|---|---|
| ALL GOOD | 20 | 80 | 2 | 35 | 63 |
| FEMALE GOOD | 21 | 79 | 2 | 37 | 61 |
| | | | | | |
| | | | | | |

| COMPARISON | GTYPE PVALUE | ALLELE PVALUE | HW PVALUE | | |
|---|---|---|---|---|---|
| ALL LIP | 0,041 | 0,3331 | 0,4801 | | |
| FEM LIP | 0,0602 | 0,1546 | 0,6376 | | |

### Extraction of Table 3

| | %FREQAA | % FREQ AG | % FREQ GG |
|---|---|---|---|
| ALL_BAD | 6 | 21 | 73 |
| ALL_GOOD | 2 | 35 | 63 |
| | | | |
| FEMALE_BAD | 5 | 20 | 75 |
| FEMALE_GOOD | 2 | 37 | 61 |

The AA genotype is 2.5 to 3 times higher in patients with bad lipids than with good lipids.

| | |
|---|---|
| Accession numbers of human Na⁺/K⁺ ATPase subunits α or β (ATP1A or B), peroxisomal farnesylated protein (PXF), and coatomer protein complex (COPA) as could be found by those skilled in the art in the Genbank database: | |

| **Gene name** | **Accession number** |
|---|---|
| ATP1A1 | NM_000701 |
| ATP1A2 | NM_000702, J05096 |
| ATP1A3 | NM_000703 |
| ATP1B1 | NM_001677,X03747 |
| ATP1B2 | NM_001678 |
| ATP1B3 | NM_001679, U51478 |
| PXF | X75535 |
| COPA | U24105 |

SEQUENCE LISTING

<110>  Bayer AG

<120>  Single Nucleotide Polymorphisms predicting Cardiovascular Disease

<130>  LeA35564

<160>  19

<170>  PatentIn version 3.1

<210>  1
<211>  20
<212>  DNA
<213>  Homo sapiens

<400>  1
tatcttcctg cagctatgac                                                    20

<210>  2
<211>  20
<212>  DNA
<213>  Homo sapiens

<400>  2
gtgtgggggt gcttgagagt                                                    20

<210> 3

<211> 23

<212> DNA

<213> Homo sapiens

<400> 3
atgggggaaa tggagggctt atc 23

<210> 4

<211> 1279

<212> DNA

<213> Homo sapiens

<400> 4
gtatctgtgt gtctgtgtct gtgtgtgtgt gagtgtgtgt ccttgaccac gtgttctcca 60

taggcgggga gtcctgcagt tactcatgga tgagtacctg tgtttgtgtc tcagcaggca 120

aatttgtaaa tgtacaagcc ttcaagcctg cacaagcata aatgtcactg tgtgggggtg 180

cttgagagtc tgtgagcctg cccttaggag ctgtgaggta tgggcttcac ggaggaagaa 240

gtcatagctg caggaagata tactgtcttg ggaccttcag ctgcatcttg acatctctgg 300

ctccccatgg aaagggcatc cctagctgag atgcaggacc tctgagaaga ggcaggggtt 360

aaaggatcag ataagccctc catttccccc atccaagtga agagagaagg aggggaggta 420

gccccctacc ctgctccatc ttagagcaag gtagacccag ctcaggaggt cttgcttggg 480

aagtgatgag accttgactt ttccagtctg tctttttccc ctagcacccc caaactcccc 540

ttaatcacca tcctaagttg ctgtgggtga tgcaatagca agatgaggag cagatctggg 600

ctgtttaaac taagaggctg ggtgaggtgg gggtatttag ggcctggagc ttagagttca 660

acctaccaac gacccctgaa gagggaaggc atctgacacc cacaacctgt tctagggatg 720

atttttcctc caatcccttc tcccctgcat ctccactgca gaggcaggct tcactgtccc 780

cccattaccc agtggctgtg aagggcagcg tgggagttgg gggaagggac gacactggtg 840

ggagggagcc cagcctgctc cagctaccac ggagaggctg agatgggggg agcgttggcg 900

gattcccagc tgcccccact ctgtcccagc ctctggcttt ctcaaaaagg actctctgtt 960

ctccttcagc attcaagacc cagagagggg gacttgtggt tgggggaggg aggagtggag 1020

ggaggttggg ggggtccttg cttcctctct ttctttcttg cctgggcagc cgctggcccc 1080

aaatctctgc aggctcctgg ctgcagagcc tgagatcttt gccaggacag gaggaggggg 1140

aaggggcagt gtgtctcaag ctctaagcct gctggagagc agggcgggag cttgggaaaa 1200

ggaggcactg cgtggagctg cttagctcag ccacaatcca gcatgccaaa gtgcatggac 1260

cagcaagttt ttaaaaagc 1279

<210> 5

<211> 1279

<212> DNA

<213> Homo sapiens

<400> 5
gtatctgtgt gtctgtgtct gtgtgtgtgt gagtgtgtgt ccttgaccac gtgttctcca 60

taggcgggga gtcctgcagt tactcatgga tgagtacctg tgtttgtgtc tcagcaggca 120

aatttgtaaa tgtacaagcc ttcaagcctg cacaagcata aatgtcactg tgtgggggtg 180

cttgagagtc tgtgagcctg cccttaggag ctgtgaggta tgggcttcac ggaggaagag 240

gtcatagctg caggaagata tactgtcttg ggaccttcag ctgcatcttg acatctctgg 300

ctccccatgg aaagggcatc cctagctgag atgcaggacc tctgagaaga ggcaggggtt 360

aaaggatcag ataagccctc catttccccc atccaagtga agagagaagg aggggaggta 420

gccccctacc ctgctccatc ttagagcaag gtagacccag ctcaggaggt cttgcttggg 480

aagtgatgag accttgactt ttccagtctg tctttttccc ctagcacccc caaactcccc 540

ttaatcacca tcctaagttg ctgtgggtga tgcaatagca agatgaggag cagatctggg 600

ctgtttaaac taagaggctg ggtgaggtgg gggtatttag ggcctggagc ttagagttca 660

acctaccaac gacccctgaa gagggaaggc atctgacacc cacaacctgt tctagggatg 720

atttttcctc caatcccttc tcccctgcat ctccactgca gaggcaggct tcactgtccc 780

cccattaccc agtggctgtg aagggcagcg tgggagttgg gggaagggac gacactggtg 840

ggagggagcc cagcctgctc cagctaccac ggagaggctg agatgggggg agcgttggcg 900

gattcccagc tgcccccact ctgtcccagc ctctggcttt ctcaaaaagg actctctgtt 960

ctccttcagc attcaagacc cagagagggg gacttgtggt tgggggaggg aggagtggag 1020

ggaggttggg ggggtccttg cttcctctct ttctttcttg cctgggcagc cgctggcccc 1080

aaatctctgc aggctcctgg ctgcagagcc tgagatcttt gccaggacag gaggaggggg 1140

aaggggcagt gtgtctcaag ctctaagcct gctggagagc agggcgggag cttgggaaaa 1200

ggaggcactg cgtggagctg cttagctcag ccacaatcca gcatgccaaa gtgcatggac 1260

cagcaagttt ttaaaaagc 1279

<210> 6

<211> 561

<212> DNA

<213> Homo sapiens

<400> 6
taaggagaag gctgtgtgca gagctgagag gggcccagtg aggtttaaag gtggagagac 60

ccaggtccaa atgtcaacac catgcctacg cccctgctaa accttctctc agtgtggggt 120

gcttgtgcag tgcctccttg catctgtgtg ttatgaatgc tctatgcccc aggtatttct 180

ttgttgtcca ctttttagca atcgtgtatt gaataccaat tatgtgtcag ctgctttata 240

tatgttcgat gtgctatctg atgtaattct gaaagagtgg tgtaatcatg cagggctgct 300

gcatgtggcc acacacgttg catcctgaat gaccccagga ggtgcatttt cataactaca 360

gtgttagtgt ctccccctgt ggttgtgcar ggcagagatt ctgtttacat tcaccatttt 420

acagttgagt acatggaggc tctcttggtt aagcaagtaa cccgaggtca cataagaagt 480

caggaggagt cagaattcca aaccagggca ggggcaatag atgagtaatg ccataaatat 540

gtgatgaggg caagtgtgac t 561

<210> 7

<211> 561

<212> DNA

<213> Homo sapiens

<400> 7
taaggagaag gctgtgtgca gagctgagag gggcccagtg aggtttaaag gtggagagac 60

ccaggtccaa atgtcaacac catgcctayg cccctgctaa accttctctc agtgtggggt 120

gcttgtgcag tgcctccttg catctgtgtg ttatgaatgc tctatgcccc aggtatttct 180

ttgttgtcca ctttttagca atcgtgtatt gaataccaat tatgtgtcag ctgctttata 240

tatgttcgat gtgctatctg atgtaattct gaaagagtgg tgtaatcatg caggctgctt 300

gcatgtggcc acacacgttg catcctgaat gaccccagga ggtgcatttt cataactaca 360

gtgttagtgt ctccccctgt ggttgtgcaa ggcagagatt ctgtttacat tcaccatttt 420

acagttgagt acatggaggc tctcttggtt aagcaagtaa cccgaggtca cataagaagt 480

caggaggagt cagaattcca aaccagggca ggggcaatag atgagtaatg ccataaatat 540

gtgatgaggg caagtgtgac t 561

<210> 8

<211> 634

<212> DNA

<213> Homo sapiens

<400> 8
agattggatg tagactcggg agacctgagt tctggctctg tcttatcaat tcaaattgtt 60

taatctctct gagcctcagt ttacccactt ggaaaagaag agttaggcca catggtctct 120

gtcatcctgg gattctgagt caactgctcc ccttcagcca gatccwaggg ggcatatccc 180

cccaacctgg gacccaggcc cccttccctt gcagcctctc tcagttgtga gtgccacagg 240

ccagaggggg ttgagaggag ccaagggggg gcatgccgtg ggacaaacgg gctgtctagg 300

gccagtggct aaaggggcgg gaggggagga gtcctcaggg atcctgtttc aacaaacgtt 360

tctttcggag gaggggaagg cgggggagag ggggagaagg acctatttaa agctaccctg 420

ttgctttggc tttctctgtc tgccagggtc tccgactgtc ccagacgggc tggtgtgggc 480

ttgggatcct cctggtgacc tctcccgcta aggtccctca gccactctgc cccaagatgg 540

gccgtggggt gagtatccct aaagagcagg ggtcgcagtc tagagggtga gggaggctgc 600

tgaggaagga tgaagtggga atgggatgtg gaag 634

<210> 9

<211> 595

<212> DNA

<213> Homo sapiens

<400> 9
ctaaggagaa ggctgtgtgc agagctgaga ggggcccagt gaggtttaaa ggtggagaga 60

cccaggtcca aatgtcaaca ccatgcctat gcccctgcta aaccttctct cagtgtgggg 120

tgcttgtgca gtgcctcctt gcatctgtgt gttatgaatg ctctatgccc caggtatttc 180

tttgttgtcc actttttagc aatcrtgtat tgaataccaa ttatgtgtca gctgctttat 240

atatgttcga tgtgctatct gatgtaattc tgaaagagtg gtgtaatcat gcagggctgc 300

tgaatgtggc cacacacgtt gcatcctgaa tgaccccagg aggtgcattt tcataactac 360

agtgttagtg tctccccctg tggttgtgca aggcagagat tctgtttaca ttcaccattt 420

tacagttgag tacatggagg ctctcttggt taagcaagta acccgaggtc acataagaag 480

tcaggaggag tcagaattcc aaaccagggc aggggcaata gatgagtaat gccataaata 540

tgtgatgagg gcaagtgtga ctttgttcct cgtgtcatga agattgagtg tggtt 595

<210> 10

<211> 595

<212> DNA

<213> Homo sapiens

<400> 10
ctaaggagaa ggctgtgtgc agagctgaga ggggcccagt gaggtttaaa ggtggagaga 60

cccaggtcca aatgtcaaca ccatgcctat gcccctgcta aaccttctct cagtgtgggg 120

tgcttgtgca gtgcctcctt gcatctgtgt gttatgaatg ctctatgccc caggtatttc 180

tttgttgtcc actttttagc aatcatgtat tgaataccaa ttatgtgtca gctgctttat 240

atatgttcga tgtgctatct gatgtaattc tgaaagagtg gtgtaatcat gcagggctgc 300

tgmatgtggc cacacacgtt gcatcctgaa tgaccccagg aggtgcattt tcataactac 360

agtgttagtg tctccccctg tggttgtgca aggcagagat tctgtttaca ttcaccattt 420

tacagttgag tacatggagg ctctcttggt taagcaagta acccgaggtc acataagaag 480

tcaggaggag tcagaattcc aaaccagggc aggggcaata gatgagtaat gccataaata 540

tgtgatgagg gcaagtgtga ctttgttcct cgtgtcatga agattgagtg tggtt 595

<210> 11

<211> 441

<212> DNA

<213> Homo sapiens


<400> 11
gcaaacacct tggctgtgga ccctacccag gtatccactc tttatgggaa acaaaagtta 60

acatcatagt gtgggaaaac taattcctga aagcctagtg cagactttgc agaatccagt 120

ggagaaatag gcaccaggtg gaaactgagc agcccctcta tgacaaggac tctatttcct 180

gagggaagtg ggaggagaga agactggggc agaagaggaa gtaaraaggt acctttttgg 240

gaacttttca tggctaccca ggccttcctc agcctctatc ctattctatc accccaagag 300

ggcctcctct tagaaggaaa gatttcccac cctgtcccca agaagttccc tgtgagaccc 360

tggaaggctg gtagtggtga gcccacaggc cctggagtgg aagggaaatc aggagcctca 420

taagactaca attaaaatgt c 441

<210> 12

<211> 476

<212> DNA

<213> Homo sapiens

<400> 12
tttgccagga caggaggagg gggaaggggc agtgtgtctc aagctctaag cctgctggag 60

agcagggcgg gagcttggga aaaggaggca ctgcgtggag ctgcttagct cagccacaat 120

ccagcatgcc aaagtgcatg gaccagcaag tttttaaaaa gcatgcattt tattccaatt 180

tttatgaaat ttatttcaca tctgaatata cagaaattcc tctgcaccct gctcttctgc 240

ccacagaccc agccctgctc ctctgtgtat gcctgggccc ccttctgtcc ctgaagttcc 300

tgagggtcac actgaagctc agccagcctg atctcttgcc tctgttccct tgattgcatt 360

tcttcctttt stcagctctg tcccaccaca gacagttagt gaatgtaaag catttcaatc 420

cactcaatta aatgaatcct cttaggtcac ctgagaaagg cagaggcaga accact 476

<210> 13

<211> 486

<212> DNA

<213> Homo sapiens

<400> 13
aattctggtt ctaccacttt ctgtgcagcc ttggcccagg tagttaatct ttcagtgcaa 60

gcagtgttgt cattcataaa atggaggtga tgcctaccat gaagtgttgc tatggaaatt 120

aactgatctg tgatattaaa gcatagtcac tggtacttgg ggttacctaa tctaaatgcc 180

agaggagtga tttaacaagg gaggcaagac cctttctttt tagccaagtg gagttagcaa 240

agtatgtttt gtatgtggaa agtctacttt tgaattgtct tcgtttctgc cttcctacta 300

atgwttttct ctctgcctgg tctaggatta acacagattc ctcagatcca gaagactgaa 360

atttcctttc gtcctaatga tcccaagagc tatgaggcat atgtactgaa catagttagg 420

ttcctggaaa agtacaaaga ttcagcccag agggatgaca tgatttttga agattgtggc 480

ggtaag 486

<210> 14

<211> 620

<212> DNA

<213> Homo sapiens

<400> 14
ctgaaatttc ctttcgtcct aatgatccca agagctatga ggcatatgta ctgaacatag 60

ttaggttcct ggaaaagtac aaagattcag cccagaggga tgacatgatt tttgaagatt 120

gtggcggtaa gtagactcat tgtagatgtc tgtggctagt tttctttctc tttaactctt 180

atttcccact tctatttttt gtttaatgat cgagagataa tcctgaaata atgaaagatt 240

gaactctggc catatttaga cacttttttt aaaaaaagca ggaagacaca atgaaaaaca 300

gtacagcytc aagtatgact gttttctgca ttggaaaggt gtcttacttt aaaacactcc 360

taggtactat tttttactat tcagagccaa gacagcaatt ccggttttct ttctcttaca 420

tccgttgaaa agtacagcta cttatttcct tatcttcccc caaaaagttc tccctataga 480

agagtgcaca gggccttctc tcatcagaaa gaccttttgg tgtgattagc tatcactgca 540

ctggcattca tgaaggagga tgtatggtca gggtatactt ttattgatcc tggttgggat 600

agataatgaa atatgtgtta 620

<210> 15

<211> 494

<212> DNA

<213> Homo sapiens

<400> 15
gaaggtccag ctgttacaaa atgaagacag acaacacaac atttactctg tggagatatc 60

ctactcatac tatgcacgtg ctgtgatttt gaacataact cgtcccaaaa acttgtcacg 120

atcatcctga ctttttaggt tggctgatcc atcaatcttg cactcaactg ttacttcttt 180

ccccagtgtt gttaggagca aagctgacct gaacagcaac caatggctgt agatacccaa 240

cctggcaata aaaggaaaat atattaattc aaactctatg gaaacttaat acttctctct 300

accaggaatt ggcttattct gtaaattttc tttttgagas tgttgcccag gctgcagtgc 360

agtggtgcaa ttatggctca ttgaagcctc aaccaatcct cccacctcag tttcctaagt 420

agctgggact ataggcacat gccgccacag cccactaatt tttaaatttt tttgtagaga 480

cagggtctcc ctgc 494

<210> 16

<211> 552

<212> DNA

<213> Homo sapiens

<400> 16
tccaaaagct ctcagaggct gcagggagag tgggtgagga gaccccagat acatggaaat 60

atctgaacag actcttaagt ctttgttgcc tgaaaacacc agaattctta gtgaagttga 120

tagcaatcat gatgtcacag ccaaggaacg ggtaaaggca atgagagaaa ggatgggaat 180

ccctgttaat gaaaaggggt gagataggtt ggaaagtaat agaaaacatt attcaaaact 240

gaccatttga aaggtcacag tgttattgtg ggtatcatca tggagaataa actgatgcag 300

rgtccgtgta caaattagat ttatgaaaag actatgaaga taaccatagg gttttccaag 360

attgggagtg ataatgattc actagtaaat gcttatatcc aagctgttaa ccagttcagt 420

ctaggcaagg aaacaaactc ctaaatgacg ctggcttttt taatctctcc taggcagtga 480

tatgacctcc caacaagaat tcacttcttg cctaaaggaa acactaagtg gattagccaa 540

aaatgccact ga 552

<210> 17

<211> 508

<212>  DNA

<213>  Homo sapiens

<400>  17
ctacctgtat ctcttgttct ctcctatttg agtttcactt ccagagaact tgttcttcag      60

caagaatgtg tcactagtaa ggacatctct agcatttctc tagccttcct tttctgctgc     120

tcaaaaataa tcgttacaaa gcttaggttt aagctgtata tgaaatattt atgcgactct     180

caaactttaa aggagttgct cctttgttcc aaaattaaat gtgttagata aatttgtgat     240

tgtatgggtg gcttcatgaa ttaagaattg aattaataca gactttttga taattgggac     300

attttcctgt gactctggaa ggtcttgtca aagccccatg aggcagttat aatatgatgt     360

taagatggct ttggggagct aggagaaaac attggcccat attgaatgac rtggagacgg     420

cagaccatac aaaaaaggac caaaagactt agaggaaatg ggtatgcaag aatgaatgtt     480

atataacact ggtaaaccca ccagctga                                        508

<210>  18

<211>  621

<212>  DNA

<213>  Homo sapiens

<400>  18
aaagtaaccc cagtcctgtc cttaagctcc agatatccca tcacaaccac cacmaagctt      60

cacatacaca tctcattact tgtgtcctcc aaatgcattc tttttgggtg atggaagtct     120

gttttaaccc tctctaggac ctgaccctga taatctctag agtaggtgga ggaatgtggg     180

tctagattat ggacgccatc ttcgtattga gaaggggaag tttgatacaa taggaatggt     240

gattgttaac ccttcacata catccagtac agaggttggg acctcttttc acgcagtaca     300

gttgtgtctc acatcaacgg aactaccaaa ccatggtgtt gggatccttt gaggtgttgt     360

ctgcagcctt ctgtcctcct aaatactcct tccttcccct agacccgaaa aatcctgtct     420

gcctgtgaga agaatcccac agatgcctac cagctcaatt atgacatgca caaacccttt     480

gacatttgtg ctgcatcata tcggcccatc taccgtggaa agccagtaga aaagtgtcca 540

ctcagtgggg cctgctattc ccctgagttc aaaggtcaaa tctgcagggt caccacagta 600

agtactgtcc tcaataaata t 621

<210> 19

<211> 544

<212> DNA

<213> Homo sapiens

<400> 19
ccggagccta agagaccgga agttcgtgtt tccaggcgct tccggaaacc gcgggagagg 60

gtcgctgacg tggaggcgtc cgaagggcag cagggtgtgt cggggctcgg attaagacat 120

cggagtcgga gacctgagag atgttaacca aattcgagac caagagcgcg cgggtcaaag 180

gtagtggaga ataaaagggg gaacatggag gttggatgtc aggagcggcg ctgacaccgg 240

ggaagagaaa atgggtagag gtgttggtcg aaatgggccc accagacgga cccggaggaa 300

gacggagggt gggagagagg ttgggcctgg ctagcggttc tccagtcccc tcttctttgg 360

tgtgtccttg ggcttcgatt tacctataaa gtggctccca cactgcaggg gattccttag 420

accccctccc tattctttct tccccctttc tctcttggct gtgctcccga tttgtrttgt 480

tctgctccaa aggccctgtg atctgttggc tttttgatga ctgtgttata tggagtggcc 540

ctgg 544

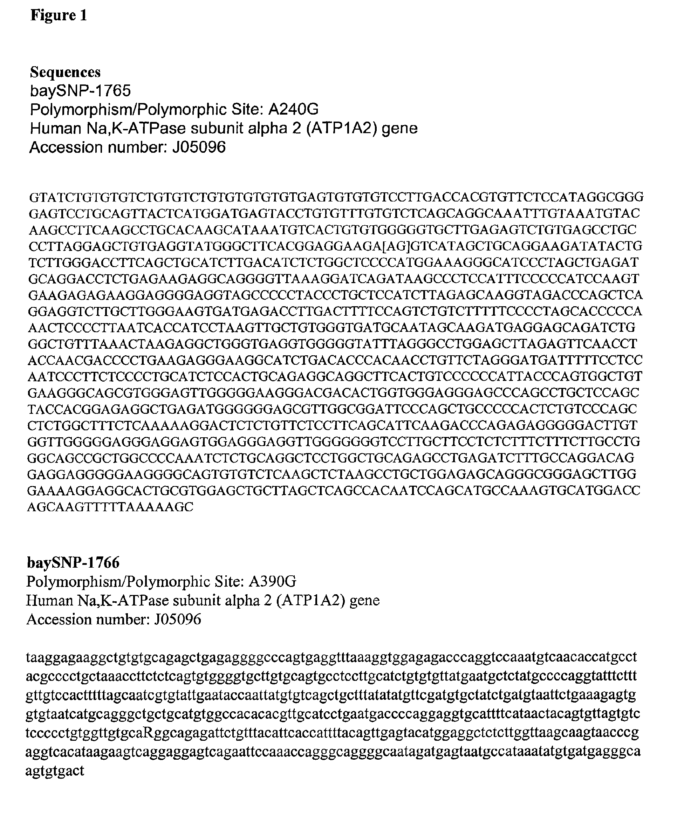

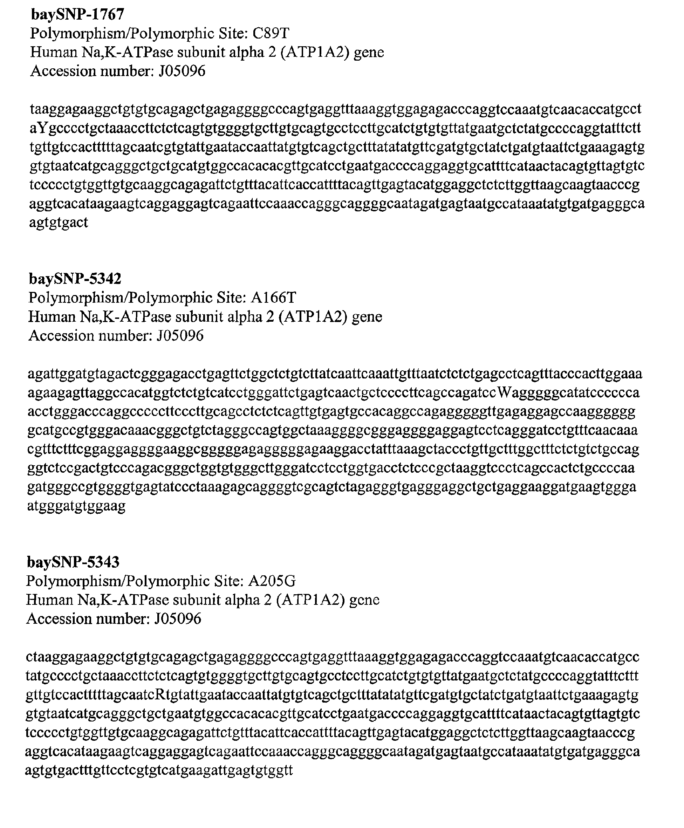

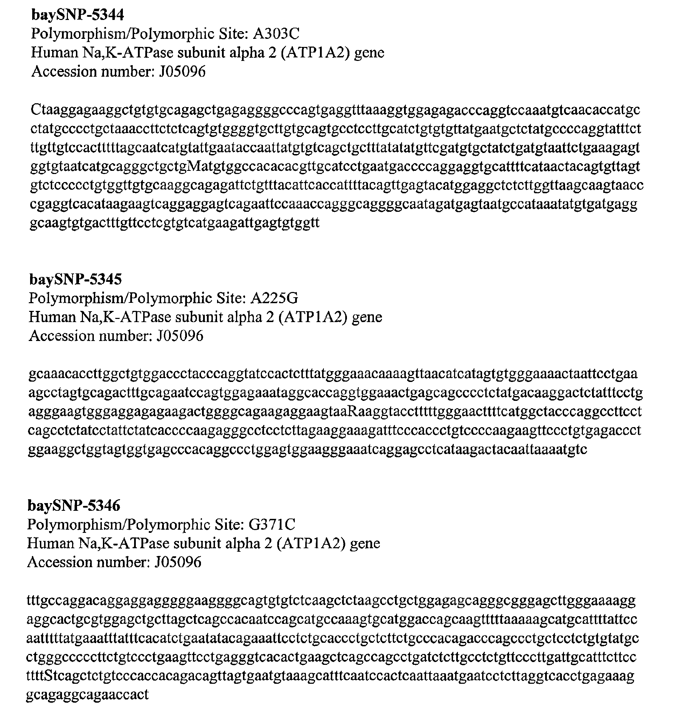

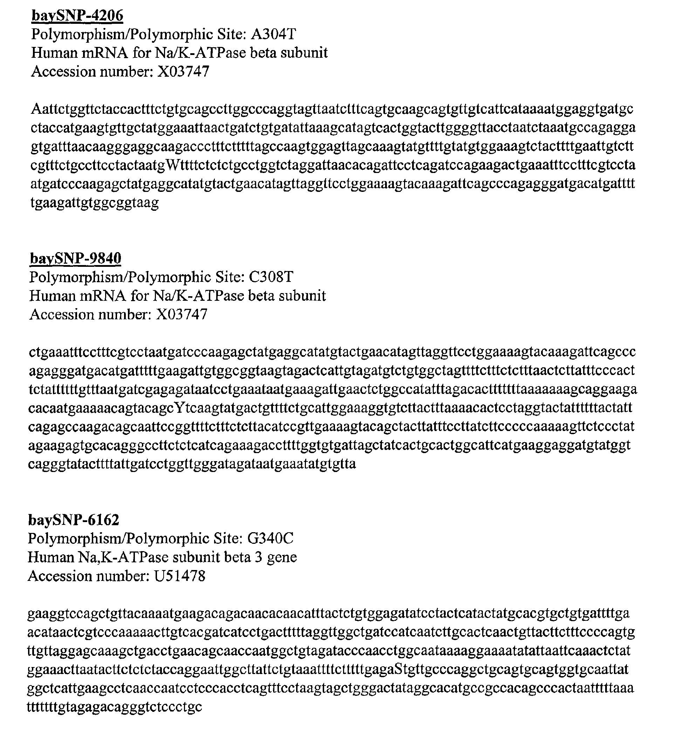

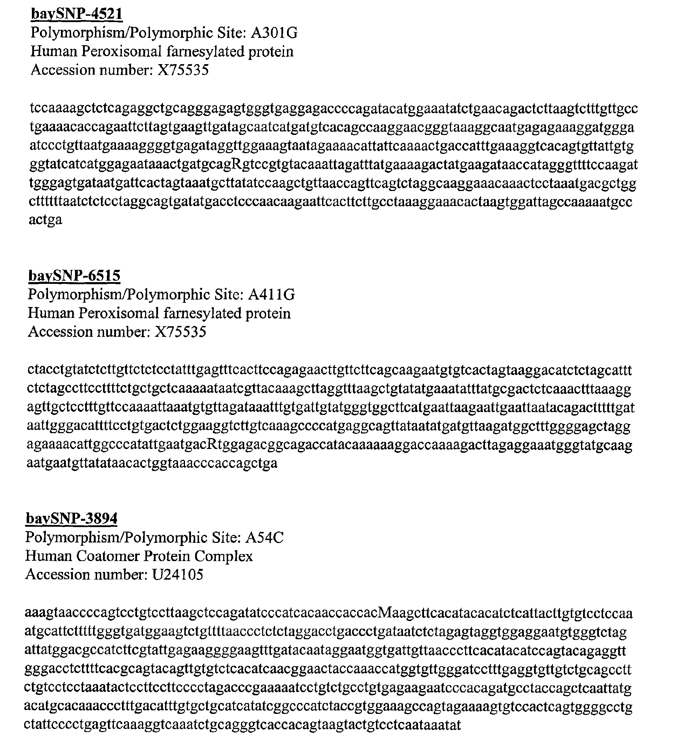

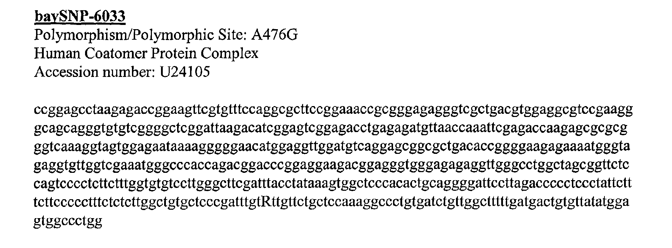

## Claims

1. An isolated polynucleotide encoding a Na⁺/K⁺ ATPase polypeptide the polynucleotide is selected from the group consisting of:
SEQ ID 4 and SEQ ID 5 (baySNP-1765) with allelic variation G in position 240 contained in a functional surrounding like full length cDNA for Na⁺/K⁺ ATPase and with or without the Na⁺/K⁺ ATPase promotor sequence; and
SEQ ID 4 and SEQ ID 5 (baySNP-1765) with allelic variation A in position 240 contained in a functional surrounding like full length cDNA for Na⁺/K⁺ ATPase and with or without the Na⁺/K⁺ ATPase promotor sequence; and
SEQ ID (baySNP-1766, -1767, -5342, -5343, -5344, -5345, -5346, -4202, -9840, and -6162) with allelic variation contained in a functional surrounding like full length cDNA for Na⁺/K⁺ ATPase and with or without the Na⁺/K⁺ ATPase promotor sequence.

2. An isolated polynucleotide encoding a peroxisomal farnesylated protein (PXF) or coatomer protein complex (COPA) polypeptide the polynucleotide is selected from the group consisting of:
SEQ ID (baySNP-4521, -6515, -3894, -6033) with allelic variation contained in a functional surrounding like full length cDNA for PXF or COPA and with or without the PXF or COPA promotor sequence.

3. An expression vector containing any polynucleotide of claim 1 or 2.

4. A host cell containing the expression vector of claim 3.

5. A substantially purified Na⁺/K⁺ ATPase, PXF, or COPA polypeptide encoded by a polynucleotide of claim 1 or 2.

6. A method for producing a Na⁺/K⁺ ATPase, PXF, or COPA polypeptide, wherein the method comprises the following steps:
a) culturing the host cell of claim 4 under conditions suitable for the expression of the Na⁺/K⁺ ATPase, PXF, or COPA polypeptide; and
b) recovering the Na⁺/K⁺ ATPase, PXF, or COPA polypeptide from the host cell culture.

7. A method for the detection of a polynucleotide of claim 1 or 2 or a Na⁺/K⁺ ATPase, PXF, or COPA polypeptide of claim 5 comprising the steps of:
contacting a biological sample with a reagent which specifically interacts with the polynucleotide or the Na⁺/K⁺ ATPase, PXF, or COPA polypeptide.

8. A method of screening for agents which regulate the activity of a Na⁺/K⁺ ATPase, PXF, or COPA comprising the steps of:
contacting a test compound with a Na⁺/K⁺ ATPase, PXF, or COPA polypeptide encoded by any polynucleotide of claim 1 or 2; and
detecting a Na⁺/K⁺ ATPase, PXF, or COPA activity of the polypeptide, wherein a test compound which increases the Na⁺/K⁺ ATPase, PXF, or COPA activity is identified as a potential therapeutic agent for increasing the activity of the Na⁺/K⁺ ATPase, PXF or COPA, and wherein a test compound which decreases the Na⁺/K⁺ ATPase, PXF, or COPA activity of the polypeptide is identified as a potential therapeutic agent for decreasing the activity of the Na⁺/K⁺ ATPase, PXF, or COPA.

9. A reagent that modulates the activity of a Na⁺/K⁺ ATPase, PXF, or COPA polypeptide or a polynucleotide wherein said reagent is identified by the method of the claim 8.

10. A pharmaceutical composition, comprising:
the expression vector of claim 3 or the reagent of claim 9 and a pharmaceutically acceptable carrier.

11. Use of the pharmaceutical composition of claim 10 for modulating the activity of a Na⁺/K⁺ ATPase, PXF, or COPA in a disease.

12. Use of claim 11 wherein the disease is atherosclerosis, ischemia/reperfusion, hypertension, restenosis, arterial inflammation, myocardial infarction, and stroke.

13. A method for determining whether a human subject has, or is at risk of developing a cardiovascular disease, comprising determining the identity of nucleotide G or A at position 240 of SEQ ID 4 and SEQ ID5 (baySNP-1765) of the Na⁺/K⁺ ATPase gene locus of the subject wherein homozygosity for the AA mutation of the gene indicates that the individual is at risk for a cardiovascular disease.

14. A kit for assessing cardiovascular status, said kit comprising:
a) sequence determination primers and
b) sequence determination reagents,
wherein said primers are selected from the group consisting of primers that hybridize to polymorphic positions in human Na⁺/K⁺ ATPase, PXF, or COPA genes; and primers that hybridize immediately adjacent to polymorphic positions in human Na⁺/K⁺ ATPase, PXF, or COPA genes.

15. A kit as defined in claims 14 detecting a combination of two or more, up to all, polymorphic sites selected from the groups of sequences as defined in claims 1 and 2.

16. A kit for assessing cardiovascular status, said kit comprising one or more antibodies specific for a polymorphic position defined in claims 1 and 2 within the human Na⁺/K⁺ ATPase, PXF, or COPA polypeptides and combinations of any of the foregoing.
